(19) Europäisches Patentamt
European Patent Office
Office européen des brevets

(11) Veröffentlichungsnummer: **0 055 428**
**B1**

(12) # EUROPÄISCHE PATENTSCHRIFT

(45) Veröffentlichungstag der Patentschrift: **14.03.84**

(21) Anmeldenummer: **81110566.7**

(22) Anmeldetag: **18.12.81**

(51) Int. Cl.[3]: **C 07 C 143/11,** C 07 D 521/00 // C07D213/20, C07D215/02, C07D235/08, C07D235/28, C07D263/56, C07D263/60, C07D277/64, C07D277/84, C07D293/12, C07D417/04

(54) **Bis-(3-sulfopropyl)-ether, Verfahren zu ihrer Herstellung und ihre Verwendung als Alkylierungsmittel für organische Basen.**

(30) Priorität: **30.12.80 DE 3049447**

(43) Veröffentlichungstag der Anmeldung: **07.07.82 Patentblatt 82/27**

(45) Bekanntmachung des Hinweises auf die Patenterteilung: **14.03.84 Patentblatt 84/11**

(84) Benannte Vertragsstaaten: **BE CH DE FR GB LI**

(56) Entgegenhaltungen:
**RECUEIL DES TRAVAUX CHIMIQUES DES PAYS-BAS, Band 52, Nr. 1038 1933 H.J. BACKER: "Acide diéthyléther-2,2'-Disulfonique", Seiten 205-207**
**CHEMICAL ABSTRACTS, Band 85, Nr. 21, 22 November 1976, Zusammenfassung Nr. 159430s, Seite 493 COLUMBUS OHIO (US)**

(73) Patentinhaber: **AGFA-GEVAERT Aktiengesellschaft, D-5090 Leverkusen 1 (DE)**

(72) Erfinder: **Kampfer, Helmut, Dr., Roggendorfstrasse 63, D-5000 Koeln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Bis-(3-sulfopropyl)-ether, Verfahren zu ihrer Herstellung und ihre Verwendung als Alkylierungsmittel für organische Basen

Die Erfindung betrifft Bis-(3-sulfopropyl)-ether, mehrere Verfahren zu ihrer Herstellung sowie ferner ihre Verwendung als Alkylierungsmittel für organische Basen.

An Bis-(sulfoalkyl)-ethern sind beschrieben Bis-(2-sulfo-ethyl)-ether (DE-PS 646 707), Bis-(4-sulfobutyl)-ether (Helberger & Lantermann, Liebigs Ann. Chem. 586 (1954) 158, 161) sowie Ethylenglykol-bis-(3-sulfopropyl)-ether (US 2 275 378) bzw. entsprechende von Polyglykolen abgeleitete Ether (BE 815 651). Diese Verbindungen werden aus entsprechenden Dihalogenalkanen durch Umsetzung mit Sulfit, aus Dimercaptoalkanen durch Oxidation mit Chlor bzw. aus Glykolen durch Umsetzung mit 1,3-Propansulton hergestellt. Sie fanden Interesse als Netzmittel, als Komponenten zur Modifizierung von Glykolkondensationsprodukten (Polyester) sowie als Entwicklungsbeschleuniger in fotografischen Entwicklern.

Bis-(4-sulfo-butyl)-ether wird verwendet als Zwischenprodukt bei der Herstellung von 1,4-Butansulton (Org. Synthesis, Coll. Vol. IV (1963), 529). Sultone wie das genannte oder auch 1,3-Propansulton, 3-Methyl-1,3-propansulton (=Isobutansulton) sind hervorragende Sulfoalkylierungsmittel für organische Basen. Doch sind Sultone cancerogene Substanzen und damit bedenklich in Umfang, Lagerung und Transport.

Es hat nicht an Versuchen gefehlt, Sultone durch andere, weniger bedenkliche Sulfoalkylierungsmittel zu ersetzen. So sind Hydroxyalkansulfonsäuren und deren Salze als Quaternierungsmittel für tertiäre heterocyclische Amine beschrieben (DE-OS 2 825 246, Research Disclosure 16374/November 1977). Desgleichen sind wäßrige Lösungen der Hydroxyalkansulfonsäuren und deren OH-funktionelle Derivate zur Herstellung von N-sulfoalkylsubstituierten Aminen vorgeschlagen worden (DD 139 577). Nachteilig an diesen auf Hydroxyalkansulfonsäuren beruhenden Verfahren sind die erforderlichen hohen Temperaturen, die Entfernung großer Wassermengen aus dem Reaktionsgemisch sowie die bei Gegenwart von 1 Mol oder mehr Wasser pro Mol Base und von starker Säure bestehende Gefahr der hydrolytischen Spaltung vieler organischer Basen.

Ferner wurden für den gleichen Verwendungszweck O-Sulfoalkylimidoester und Uroniumsalze (DE-OS-2 909 200; Research Disclosure 18040/April 1979) beschrieben. Imidoester und Uroniumsalze sind jedoch relativ teuer und außerdem nur zur Alkylierung tertiärer Basen geeignet. Damit ist die Anwendungsbreite im Vergleich zu den Sultonen stark eingeschränkt. Verbesserte, als Sulton-Ersatz verwendbare Sulfoalkylierungsmittel sind die in den deutschen Patentanmeldungen P 3 004 692.5 und P 2 010 427.9 beschriebenen Hydroxyalkansulfonsäuresulfoalkylester. Nachteilig ist hier jedoch der Restwassergehalt von 1 – 1,5 Mol in diesen Verbindungen sowie die Art der Herstellung.

Aus diesen Gründen besteht erhebliches Interesse an billigen, physiologisch unbedenklichen Ersatzstoffen für Sultone, insbesondere 1,3-Propansultone, die die genannten Nachteile nicht besitzen.

Gegenstand der Erfindung sind Bis-(3-sulfopropyl)-ether der allgemeinen Formel I

$$X^1O_3S-\underset{R^1}{\underset{|}{CH}}-\underset{R^2}{\underset{|}{CH}}-\underset{R^3}{\underset{|}{CH}}-O-\underset{R^3}{\underset{|}{CH}}-\underset{R^2}{\underset{|}{CH}}-\underset{R^1}{\underset{|}{CH}}-SO_3X^2 \qquad (I)$$

worin bedeuten

$X^1$, $X^2$ H oder das Kation einer anorganischen oder organischen Base,
$R^1$, $R^2$, $R^3$ H oder Methyl.

Besonders geeignet sind Bis-(3-sulfopropyl)-ether der Formel I, in der $R^2$ und $R^3$ für H stehen.

Die durch $X^1$ und $X^2$ dargestellten Kationen anorganischer Basen sind $NH_4^{\oplus}$ -Ionen oder Metallkationen, insbesondere Alkalimetallkationen wie $Na^{\oplus}$ und $K^{\oplus}$. Bevorzugte Beispiele von Kationen organischer Basen entsprechen der Formel $NR_4$ , worin die vorhandenen Reste R gleich oder verschieden sind und für H, Alkyl mit bis zu 4 C-Atomen oder Benzyl stehen und wobei mindestens einer der Reste R nicht H bedeutet.

Ein weiterer Gegenstand der Erfindung ist ein Verfahren zur Herstellung der Bis-(3-sulfopropyl)-ether der allgemeinen Formel I, dadurch gekennzeichnet, daß ein Bis-(3-halogenpropyl)-ether der Formel II

$$Hal-\underset{R^1}{\underset{|}{CH}}-\underset{R^2}{\underset{|}{CH}}-\underset{R^3}{\underset{|}{CH}}-O-\underset{R^3}{\underset{|}{CH}}-\underset{R^2}{\underset{|}{CH}}-\underset{R^1}{\underset{|}{CH}}-Hal$$

worin $R^1$, $R^2$ und $R^3$ die oben angegebene Bedeutung haben und Hal für ein Halogenatom, insbesondere für Chlor oder Brom steht, mit einem Salz, vorzugsweise Ammonium- oder Alkalisalz der schwefeligen Säure umgesetzt wird.

Ähnliche Umsetzungen zur Herstellung von Bis-(2-sulfoethyl)-ether (DE-PS-646 707) und Bis-(4-sulfobutyl)-ether (Helberger & Lantermann, loc. cit.) wurden bereits beschrieben.

Ein weiterer Gegenstand der Erfindung ist ein Verfahren zur Herstellung der Bis-(3-sulfopropyl)-ether der Formel I durch Oxidation der entsprechenden Bis-(3-mercaptopropyl)-ether bzw. der davon abgeleiteten reinen S-substituierten Derivate wie z. B. Bis-(3-thioureidopropyl)-ether oder Bis-(3-thiocyanato-propyl)-ether mit Oxidationsmitteln wie Salpetersäure, Chlor, Brom oder Wasserstoffsuperoxid, ggf. in Gegenwart eines Katalysators, mit Permanganat, Bichromat oder Chlorbleichlauge. Ähnliche Umsetzungen sind im Fall des Bis-(2-mercaptoethyl)-ethers und des Bis-(2-thioureido-ethyl)-ethers (Backer, Rec. Trav. Chim. Pays-Bas 54 (1935) 207) sowie des Sulfochlorids des Ethylenglykol-bis-(2-sulfoethyl)-ethers (US 2 275 378) beschrieben. Bis-(3-mercaptopropyl)-ether ist aus 3,3'-Dichlorpropylether und Ammoniumhydrogensulfid erhältlich (Simpson, Canad. J. Res. (B) 25 (1947), 25).

Ein weiterer Gegenstand der Erfindung ist schließlich die Verwendung der Bis-(3-sulfopropyl)ether der Formel I als Quaternierungsmittel zur Herstellung von 3-Sulfopropylquartärsalzen tertiärer Amine. Die Umsetzung der Bis-(3-sulfopropyl)-ether der Formel I mit den zu quaternierenden tertiären Aminen wird zweckmäßigerweise bei erhöhter Temperatur, z. B. bei einer Temperatur zwischen 80 und 200° C, vorzugsweise zwischen 120 und 180° C, durchgeführt. Im allgemeinen läuft die Reaktion innerhalb des zuletzt genannten Temperaturbereichs glatt ab. Es kann jedoch, etwa bedingt durch die Natur eines verwendeten Lösungsmittels, auch außerhalb dieses Temperaturbereiches gearbeitet werden.

An tertiären Aminen sind geeignet prinzipiell alle Derivate des Ammoniak ($NH_3$), in denen jedes der drei Wasserstoffatome substituiert ist, z. B. durch ein Kohlenstoffatom eines Alkyl- oder Arylrestes oder durch ein Kohlenstoffatom oder ein Heteroatom eines heterocyclischen Ringes, wobei insbesondere das Stickstoffatom des tertiären Amins in den heterocyclischen Ring einbezogen sein kann. So sind besonders bevorzugt geeignet heterocyclische Basen der allgemeinen Formel III

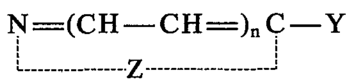

(III)

worin Z die zur Ergänzung einer heterocyclischen Gruppe mit mindestens einem 5- oder 6gliedrigen heterocyclischen Ring erforderlichen Atome bedeuten; der Heteroring kann dabei ankondensierte Benzol-, Naphthalin- oder auch heterocyclische Ringe enthalten, die auch weiter substituiert sein können; in Frage kommen die aus der Klasse der Cyaninfarbstoffe bekannten Heterocyclen wie z. B.: Pyrrolin (z. B. 4,4-Dimethyl-pyrrolin), Oxazolin (z. B. 4,4-Dimethyloxazolin), Thiazolin (z. B. 5-Methylthiazolin), Selenazolin, Indolin (z. B. 3,3-Dimethylindolin, 3,3-Dimethyl-5-methoxyindolin, 3,3-Dimethyl-5-diethylaminoindolin), Benzimidazol (z. B. 1-Ethyl-5-trifluormethylbenzimidazol, 1-Methyl-5-chlorbenzimidazol, 1-Ethyl-5,6-dichlor-benzimidazol, 1-Ethyl-5-cyanbenzimidazol, 1-Methyl-5-carbethoxybenzimidazol, 1-Ethyl-5-acetylbenzimidazol, 1-Methyl-benzimidazol-5-sulfonsäurepyrrolidid, 1-Ethylbenzimidazol-5-sulfonsäuredimethylamid, 1-Ethyl-5-phenylthiobenzimidazol, 1-Methyl-5-methyl-thiobenzimidazol, 1-Methyl-5-chlor-6-methyl-thiobenzimidazol), Oxazol (z. B. 4-Methyloxazol, 4,5-Diphenyloxazol, 4-Methyl-5-carbethoxyoxazol, Benzoxazol, 5-Chlorbenzoxazol, 5-Phenylbenzoxazol, 6-Methoxybenzoxazol, 5-Methoxybenzoxazol, 5-Methyl-6-methoxybenzoxazol, 5-Brombenzoxazol, 5-Iodbenzoxazol, Naphtho[2,1-d]oxazol, Naphtho[1,2-d]oxazol, Naphtho-[2,3-d]oxazol, 4,5,6,7-Tetrahydrobenzoxazol, Benzofuro [2,3-f]benzoxazol), Thiazol (z. B. 4-Methylthiazol, 4-Phenylthiazol, 4-Methylthiazol-5-acrylsäureethylester, Benzthiazol, 5-Methylbenzthiazol, 6-Methylbenzthiazol, 5-Chlorbenzthiazol, 5-Methoxybenzthiazol, 6-Methoxybenzthiazol, 5,6-Dimethylbenzthiazol, 5,6-Dimethoxybenzthiazol, 5-Methyl-6-methoxybenzthiazol, 5-Brombenzthiazol, 5-Phenylbenzthiazol, 6-Methylthiobenzthiazol, 6-Dimethylamino-benzthiazol, 5-Chlor-6-methoxybenzthiazol, 5,6-Methylendioxybenzthiazol, 6-$\beta$-Cyanethoxybenzthiazol, 5-Carbomethoxybenzthiazol, 5-Nitrobenzthiazol, 5-Phenylthiobenzthiazol, 5-Thienylbenzthiazol, 6-Hydroxybenzthiazol, 4,5,6,7-Tetrahydrobenzthiazol, 4-Oxo-4,5,6,7-tetrahydrobenzthiazol, Naphtho-[2,1-d]-thiazol, Naphtho[1,2-d]-thiazol, 4,5-Dihydronaphtho[1,2-d]-thiazol, 5-Methoxynaphtho-[1,2-d]-thiazol, 5,7,8-Trimethoxy-naphtho[1,2-d]-thiazol), Selenazol, (z. B. Benzselenazol, 5-Methylbenzselenazol, 5,6-Dimethylbenzselenazol, 5-Methoxybenzselenazol, 5-Methyl-6-methoxybenzselenazol, 5,6-Dimethoxybenzselenazol, 5,6-Methylendioxybenzselenazol, 6-Methylbenzselenazol, Naphtho[1,2-d]-selenazol), 1,3,4-Oxadiazol (z. B. 5-Methyl-1,3,4-oxadiazol, 5-Phenyl-1,3,4-oxadiazol), 1,3,4-Thiadiazol (z. B. 5-Methyl-1,3,4-thiadiazol, 2,5-Bis-methylthio-1,3,4-thiadiazol, 5-Benzylthio-1,3,4-thiadiazol, 2-Mercapto-5-methylthio-1,3,4-thiadiazol, 5-Carbethoxymethylthio-1,3,4-thiadiazol), Pyridin (z. B. 2-Methylpyridin, 4-Methylpyridin), Pyrimidin (z. B. 2-Methyl-4-methyl-thiopyrimidin), Chinolin (z. B. 6-Methylchinolin, 6-Methoxychinolin, 8-Chlorchinolin, 6-Fluorchinolin, 5,6-Benzochinolin, 6,7-Benzochinolin), Imidazol-[4,5-b]-chinoxalin.

n = 0 oder 1

Y bedeutet Wasserstoff, Halogen, eine gesättigte oder ungesättigte aliphatische Gruppe, insbesondere mit bis zu 6 C-Atomen, ggf. substituiert, z. B. Methyl, Ethyl, Allyl, Cyanalkyl, Halogenalkyl, Alkoxyalkyl, Alkoxy, z. B. Carboxyalkoxy, Alkylthio, z. B. Carboxyalkylthio, Sulfoalkylthio, Carbalkoxyalkylthio oder Mercapto.

Y kann beispielsweise weiterhin eine Methinkette mit 1,3 oder 5 Methingruppen bedeuten, an deren Ende sich meist über die 2-Stellung angeknüpft eine N-alkylierte heterocyclische Base befindet, wie sie in der Chemie der Cyaninfarbstoffe bekannt sind. Hierzu sei verwiesen auf F. M. Hamer, »The Cyanine Dyes and Related Compounds«, (1964), Interscience Publishers John Wiley and Sons. Verbindungen der Formel III, in der Y in der erwähnten Weise definiert ist, werden als »entquaternierte Cyaninfarbstoffe« bezeichnet. Falls solche entquaternierte Cyaninfarbstoffe nach den Verfahren der Erfindung umgesetzt werden, sind die unmittelbaren Verfahrensprodukte ohne weitere Umsetzung als Sensibilisierungsfarbstoffe geeignet.

Die Quaternierungsaktion wird im allgemeinen ohne Lösungsmittel durchgeführt, kann jedoch auch in Gegenwart eines geeigneten Lösungsmittels vorgenommen werden. Als Lösungsmittel eignen sich alle in bezug auf die erfindungsgemäße Umsetzung inerten Lösungsmittel mit hohem Lösungsvermögen für die Reaktionspartner, wie z. B. Phenol oder m-Kresol sowie m-Xylol, Chlorbenzol, Anisol.

Die erfindungsgemäßen Quaternierungsreaktionen können unter Abspaltung von Wasser erfolgen. In den meisten Fällen werden die erfindungsgemäßen Bis-(3-sulfopropyl)-ether mit den tertiären Basen im Molverhältnis 1 : 2 umgeszt, wobei pro Mol Base 0,5 Mol Wasser gebildet wird. Die Umsetzung kann aber auch in anderen Molverhältnissen — z. B. 1 : 1 — durchgeführt werden, in welch letzteren Fall kein Wasser freigesetzt wird. Vorteilhafterweise wird das ggf. entstehende Wasser aus dem Reaktionsgefäß entfernt, beispielsweise durch

1) Arbeiten unter Vakuum,
2) Einleiten eines trockenen Inertgases, z. B. Stickstoff,
3) Gegenwart eines wasserentziehenden Mittels, entweder im Reaktionsgefäß (z. B. Anhydrid einer organischen Säure oder $P_4O_{10}$) oder in einer mit dem Reaktionsgefäß verbundenen Vorlage (z. B. $P_4O_{10}$, konzentrierte Schwefelsäure, NaOH oder andere Trockenmittel),
4) Verdampfen und Ausfrieren in einer Gefrierapparatur.

Die nach dem Verfahren der Erfindung hergestellten Sulfobetaine tertiäre Amine sind insbesondere solche der folgenden Formel IV

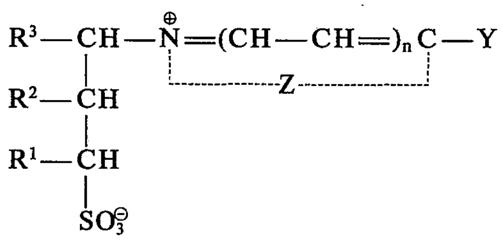

(IV)

worin Y, Z und $R^1$, $R^2$, $R^3$ die bereits angegebene Bedeutung haben. Diese Verbindungen finden unterschiedliche Verwendung. Sie sind beispielsweise als Leitsalze in der Galvanotechnik geeignet. Des weiteren handelt es sich bei diesen Verbindungen bei geeigneter Bedeutung von Y, d. h. wenn Y, wie bereits erwähnt, eine Methinkette mit 1, 3 oder 5 Methingruppen, an deren Ende sich eine N-alkylierte heterocyclische Base befindet, bedeutet, um die Endprodukte einer Cyaninfarbstoffsynthese. Derartige Verbindungen finden unmittelbar Verwendung für die spektrale Sensibilisierung von lichtempfindlichen Silberhalogenidemulsionen. Aber auch als Zwischenprodukte bei der Synthese von Polymethinfarbstoffen sind die nach dem Verfahren gemäß der Erfindung hergestellten Verbindungen wichtig. So werden beispielsweise die nach dem erfindungsgemäßen Verfahren hergestellten Sulfopropylquartärsalze heterocyclischer Basen mit Vorteil nicht isoliert, sondern nach Beendigung der Quaternierungsreaktion ohne weitere Reinigungsoperation in bekannter Weise weiter zu Polymethinfarbstoffen umgesetzt.

Die Verwendung von in wäßriger Lösung vorliegenden Hydroxyalkansulfonsäuren oder deren OH-funktionellen Derivaten der Formel $R\text{-}O\text{-}(CH_2)_n\text{-}SO_3H$ als Sulfoalkylierungsmittel ist in DD 139 577 beschrieben, wobei R unter anderem auch Sulfoalkyl bedeuten soll, jedoch in dieser Form nicht durch konkrete Beispiele belegt ist. Der Vorteil der Verwendung der Quaternierungsmittel in wäßriger Lösung wird nach DD 139 577 darin gesehen, daß auf die Isolierung und Reindarstellung der Quaternierungsmittel verzichtet werden kann. Die für die Entfernung des überschüssigen Wassers erforderlichen Reaktionsbedingungen wirken sich aber in vielen Fällen ungünstig aus, sofern hydrolyseempfindliche Basen sulfoalkyliert werden sollen. Die Tatsache, daß die erfindungsgemäßen Bis-(3-sulfopropyl)-ether anders als viele andere Sulfoalkylierungsmittel in nahezu wasserfreier Form isoliert und für die Quaternierung eingesetzt werden können, erweist sich daher als vorteilhaft, da für die Quaternierungsreaktion mildere Reaktionsbedingungen gewählt und eingehalten werden können.

Die erfindungsgemäßen Verfahren und seine Varianten werden durch die folgenden Beispiele näher erläutert:

## Beispiel 1

### Bis-(3-sulfopropyl)-ether (»Ether 1«)

a) 286 g 3-Chlorpropanol wurden mit 58 g konzentrierter Schwefelsäure versetzt und am Wasserabscheider 4 h auf 140°C erhitzt. Nach Aufarbeitung mit Toluol/Eiswasser wurden 101,8 g Bis-(3-chlorpropylether ($Kp_{21\ mb}$ 95–105° C; $n_D^{21}$ 1,4569)

b) 85,5 g des Bis-(3-chlorpropyl)-ethers wurden in 450 ml Wasser unter Zusatz von 189 g Natriumsulfit unter starkem Rühren und unter Stickstoff 60 h zum Sieden erhitzt. Danach wurde mit 86 ml konzentrierter Salzsäure angesäuert und in der Wärme das sich entwickelnde Schwefeldioxid vertrieben. Geringe Mengen Sulfat wurden mit Bariumchlorid entfernt. Die Reaktionslösung wurde an einem Kationenaustauscher von Kationen befreit, im Vakuum eingeengt und an einer Gefrierapparatur (–38° C) bei 40° C getrocknet.
Ausbeute: 123 g
Die Verbindung liegt als nahezu wasserfreies Öl vor und wird durch die chemischen Verschiebungen im $^{13}$-C-NMR-Spektrum in $D_2O$ (mit Dioxan als innerem Standard) charakterisiert:
$^{13}$C-shifts (rel. zu TMS = O); 69,49; 48,83; 25,13 ppm; Intensitätsverhältnis 1 : 1 : 1.

## Beispiel 2

### Bis-(3-sulfobutyl)-ether (»Ester 2«)

Wie in Beispiel 1 unter b) beschrieben, wurde Bis-(3-chlorbutyl)-ether, der nach den Angaben von US 2 024 749 hergestellt werden kann, mit Natriumsulfit umgesetzt und aufgearbeitet. Man erhielt den Sulfobutylether als dickflüssiges Öl.
$^{13}$C-NMR-shifts (rel. zu TMS = O): 68,53; 53,58; 31,68; 15,28 ppm; Intensitätsverhältnis 1 : 1 : 1 : 1.

## Beispiel 3

### Anhydro-2-methyl-3-(3-sulfopropyl)-5-phenyl-benzoxazoliumhydroxid, Fp. 286–288° C

13,1 g Bis-(3-sulfopropyl)ether (im folgenden »Ether 1« bezeichnet) werden mit 21 g 2-Methyl-5-phenylbenzoxazol zusammengeschmolzen und 2,5 h auf 165° C im Ölbad erwärmt. Nach dem Abkühlen wird das Reaktionsprodukt mit Aceton und Ethanol aufgearbeitet und getrocknet.
Ausbeute: 24 g = 72% d. Theorie.

## Beispiel 4

### Anhydro-2-methyl-3-(3-sulfopropyl)-naphtho[1,2 : d]oxazoliumhydroxid, Fp. 273–274° C

9,1 g 2-Methylnaphtho[1,2 : d]oxazol wurden mit 6,6 g »Ether 1« 4 h auf 160° C erhitzt, wobei nach 2 h 30 ml getrocknetes Anisol zugegeben wurde. Aufarbeitung mit Aceton, dann Propanol.
Ausbeute: 12,3 g = 81%.

## Beispiel 5

### Anhydro-2,5,6-trimethyl-3-(3-sulfobutyl)-benzthiazoliumhydroxid, Fp. 287–288° C

71 g 2,5,6-Trimethylbenzthiazol wurden unter Zusatz von 50 ml m-Kresol mit 58 g »Ether 2« 6 h bei 180° C gerührt. Nach dem Abkühlen wurde mit Aceton verrührt und das Quartärsalz aus Isopropanol unter Zusatz von Methanol umkristallisiert.
Ausbeute: 81,4 g = 65%.

## Beispiel 6

### Anhydro-2-methyl-3-(3-sulfopropyl)-naphtho[1,2 : d]thiazoliumhydroxid, Fp. 276–277° C

10 g »Ether 1« zusammen mit 15 g 2-Methylnaphtho [1,2 : d]thiazol 6 h auf 180–190°C erhitzt unter einem schwachen Strom von getrocknetem Stickstoff. Nach dem Abkühlen wird aus Ethanol umkristallisiert.
Ausbeute: 12 g = 50%.

## Beispiel 7

Anhydro-2-methyl-3-(3-sulfopropyl)-5-methoxybenzselenazoliumhydroxid, F. 298–300° C

Analog Beispiel 4 aus 11,3 g 2-Methyl-5-methoxy-benzselenazol und 7 g »Ether 1« bei 170° C. Ausbeute: 11,8 g = 70%.

## Beispiel 8

Anhydro-2-methyl-3-(3-sulfobutyl)-benzthiazoliumhydroxid, Fp. 275–278° C

Aus 2-Methylbenzthiazol und »Ether 2« im Molverhältnis 2 : 1 bei 175° C in 4 h analog Beispiel 4. Ausbeute: 68%.

## Beispiel 9

Anhydro-1,2,-dimethyl-5,6-dichlor-3-(3-sulfopropyl)-benzimidazoliumhydroxid, Fp. > 340 C

13,5 g »Ether 1« wurden mit 21,5 g 1,2-Dimethyl-5,6-dichlorbenzimidazol und 15 g Phenol 8 h auf 190° C Ölbadtemperatur erhitzt. Nach dem Abkühlen wurde das Reaktionsprodukt mit Ethanol verrieben und abgesaugt.
Ausbeute: 30 g = 89% d. Theorie.

## Beispiel 10

Anhydro-1,2-dimethyl-5-cyan-3-(3-sulfopropyl)- benzimidazoliumhydroxid, Fp. > 340° C

Analog Beispiel 9 aus 1,2-Dimethyl-5-cyanbenzimidazol in 83%iger Ausbeute.

## Beispiel 11

Anhydro-2-methyl-1-(3-sulfopropyl-chinoliniumhydroxid, Fp. 259–260° C

Aus Chinaldin und »Ether 1« analog Beispiel 8 (7 h) in 43%iger Ausbeute.

## Beispiel 12

Anhydro-2,5-dimethyl-6-methoxy-3-(3-sulfobutyl)-benzselenazoliumhydroxid. Fp. 290–292° C

Aus 2,5-Dimethyl-6-methoxybenzselenazol und »Ether 2« analog Beispiel 4; 65% Ausbeute.

## Beispiel 13

Anhydro-1-(3-sulfopropyl)-pyridiniumhydroxid, Fp. 274–277° C.

Aus Pyridin und »Ether 1« durch 6stündiges Erhitzen auf 175° C Kristallisation aus Methanol. Ausbeute: 48%.

## Beispiel 14

Anhydro-1-methyl-2-(3-sulfopropylthio)-3-(3-sulfopropyl)-5,6-dichlorbenzimidazol
Fp. 246–247° C

a) 1-Methyl-2-(3-sulfopropylthio)-5,6-dichlorbenzimidazol, Fp. 268–272° C.
18,6 g 1-Methyl-5,6-dichlor-2-(3 H)-benzimidazolthion (Fp. 230–232° C) wurden in 7 g m-Kresol mit 11,4 g »Ether 1« 4 h auf 140° C erwärmt. Nach Abkühlen auf 80° C wurde Methanol zwecks Kristallisation dazugegeben.
Ausbeute: 25,6 g = 90%.

b) Anhydro-1-methyl-2-(3-sulfopropyl-thio)-3-(3-sulfopropyl)-5,6-dichlorbenzimidazol durch Erhitzen von 1-Methyl-2-(3-sulfopropylthio)-5,6-dichlorbenzimidazol (Beispiel 14a) mit »Ether 1« im Molverhältnis 1 : 1 auf 190° C (4 h) in 89%iger Ausbeute.

### Beispiel 15

5-(3-Sulfopropyl-5-methylthio-1,3,4-thiadiazolyl-2-iden)-3-äthyl-rhodanin, Pyridinsalz. Fp. 206 – 208° C. 9 g 2,5-Dimethylthio-1,3,4-thiadiazol wurden mit 14 g »Ether 1« und 5 g Phenol 2 h auf 140° C erhitzt. Nach Abkühlen auf 80° C gab man 8 g 3-Ethylrhodanin und 25 ml Pyridin hinzu und rührte 5 h. Der ausgefallene Farbstoff wurde aus Propanol umkristallisiert.
Ausbeute: 5,5 g = 22%.

### Beispiel 16

5,5'-Dichlor-3,3'-bis-(3-sulfopropyl)-thiacyanin, Triethylaminsalz, Fp. 326° C

a) 10,1 g 5-Chlor-2-(3 H)-benzthiazolthion und 14 g »Ether 1« in 5 ml m-Kresol wurden 5 h auf 140° C erhitzt. Nach Abkühlen auf 100° C gab man eine Lösung von 15,3 g Anhydro-2-methyl-3-(3-sulfopropyl)-5-chlorbenzthiazoliumhydroxid in 70 ml m-Kresol und nach weiterem Abkühlen auf 30° C 25 ml Triethylamin hinzu. Nach 40 min Rühren wurde der Farbstoff mit Isopropanol ausgefällt.
Ausbeute: 18,5 g = 53%.
b) analog kann der Farbstoff auch aus 2-Methylthio-5-chlorbenzthiazol anstelle des 5-Chlor-2-(3 H)-benzthiazolthions erhalten werden.

## Patentansprüche

1. Bis-(3-sulfopropyl)-ether der Formel I

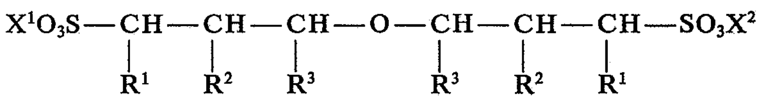

(I)

worin bedeuten

$X^1$, $X^2$ H oder das Kation einer anorganischen oder organischen Base
$R^1$, $R^2$, $R^3$ H oder Methyl

2. Bis-(3-sulfopropyl)-ether der Formel I

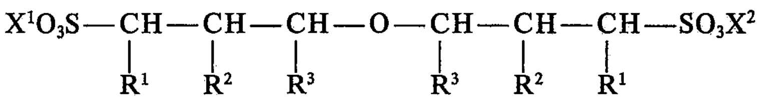

(I)

worin bedeuten

$X^1$, $X^2$ H, $NH_4^{\oplus}$, ein Alkalimetallkation oder ein Kation der Formel $NR_4^{\oplus}$, worin die vorhandenen Reste R gleich oder verschieden sind und für H, Alkyl mit bis zu 4 C-Atomen oder Benzyl stehen und wobei mindestens einer der Reste nicht H bedeutet,
$R^1$ H oder Methyl, und
$R^2$, $R^3$ H.

3. Bis-(3-sulfopropyl)-ether.
4. Bis-(3-sulfobutyl)-ether.
5. Verfahren zur Herstellung von Bis-(3-sulfopropyl)-ethern der Formel I

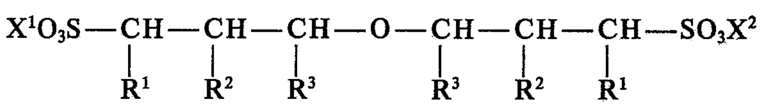

(I)

worin bedeuten

$X^1$, $X^2$ H oder das Kation einer anorganischen oder organischen Base

$R^1$, $R^2$, $R^3$ H oder Methyl,

dadurch gekennzeichnet, daß ein Bis-(3-halogenpropyl)-ether der Formel II

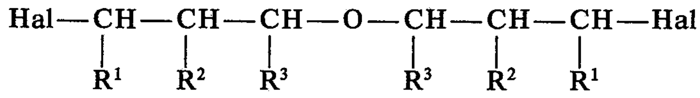

worin $R^1$, $R^2$ und $R^3$ die angegebene Bedeutung haben und Hal für ein Halogenatom steht, mit einem Salz der schwefligen Säure umgesetzt wird.

6. Verfahren zur Herstellung von Bis-(3-sulfopropyl)-ethern der Formel I

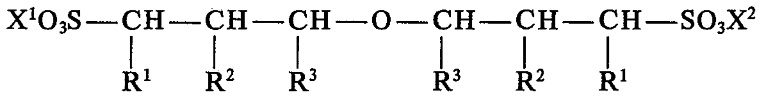

(I)

worin bedeuten

$X^1$, $X^2$ H oder das Kation einer anorganischen oder organischen Base
$R^1$, $R^2$, $R^3$ H oder Methyl,

dadurch gekennzeichnet, daß entsprechende Bis-(3-mercaptopropyl)-ether oder davon abgeleitete S-substituierte Derivate oxidiert werden.

7. Verfahren nach Anspruch 6, dadurch gekennzeichnet, daß als Oxidationsmittel Salpetersäure, Chlor, Brom, $H_2O_2$, Permanganat, Bichromat oder Chlorbleichlauge verwendet wird.

8. Verwendung von Bis-(3-sulfopropyl)-ethern der Formel I

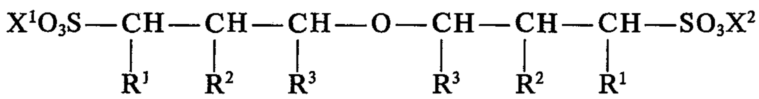

(I)

worin bedeuten

$X^1$, $X^2$ H oder das Kation einer anorganischen oder organischen Base
$R^1$, $R^2$, $R^3$ H oder Methyl,

zur Sulfoalkylierung tertiärer Amine.

9. Verwendung von Bis-(3-sulfopropyl)-ethern der Formel I

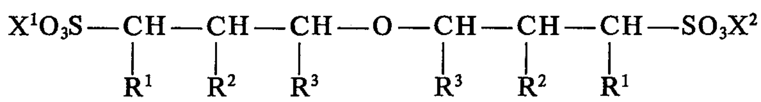

(I)

worin bedeuten

$X^1$, $X^2$ H oder das Kation einer anorganischen oder organischen Base
$R^1$, $R^2$, $R^3$ H oder Methyl,

zur Sulfoalkylierung heterocyclischer Basen der Formel III

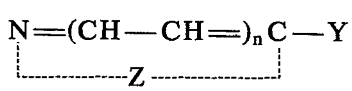

(III)

worin bedeuten

Z die zur Vervollständigung einer heterocyclischen Gruppe mit mindestens einem 5- oder 6gliedrigen heterocyclischen Ring erforderlichen Atome
n 0 oder 1
Y H, Halogen, eine gesättigte oder ungesättigte aliphatische Gruppe, Alkoxy, Alkylthio oder Mercapto.

**Claims**

1. Bis-(3-sulphopropyl)-ethers of the formula I

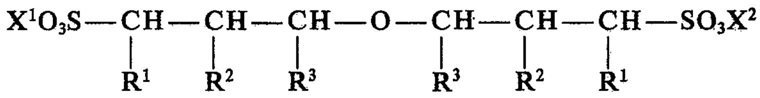

(I)

wherein

$X^1$ and $X^2$ represent H or the cation of an inorganic or organic base, and
$R^1$, $R^2$ and $R^3$ represent H or methyl.

2. Bis-(3-sulphopropyl)-ethers of the formula I

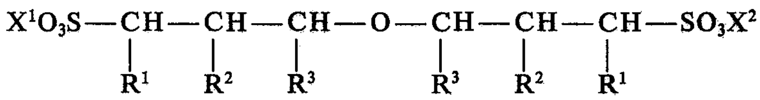

(I)

wherein

$X^1$ and $X^2$ represent H, $NH_4^{\oplus}$, an alkali metal cation or a cation of the formula $NR_4^{\oplus}$ wherein the groups R present are the same or different and represent H, alkyl having up to 4 carbon atoms or benzyl, provided that at least one of the groups R does not represent H,
$R^1$ represents H or methyl, and
$R^2$ and $R^3$ represent H.

3. Bis-(3-sulphopropyl)-ether.
4. Bis-(3-sulphobutyl)-ether.
5. Process for the preparation of bis-(3-sulphopropyl)-ethers of the formula I

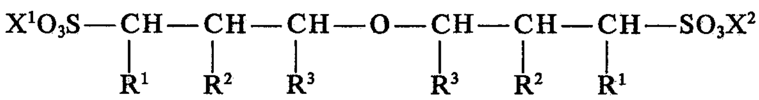

(I)

wherein

$X^1$ and $X^2$ represent H or the cation of an inorganic or organic base, and
$R^1$, $R^2$ and $R^3$ represent H or methyl,

characterised in that a bis-(3-halogen-propyl)-ether of the formula II

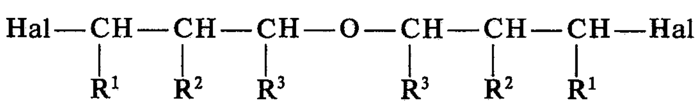

wherein $R^1$, $R^2$ and $R^3$ have the meaning indicated and Hal represents a halogen atom, is reacted with a salt of sulphurous acid.

6. Process for the preparation of bis-(3-sulphopropyl)-ethers of the formula I

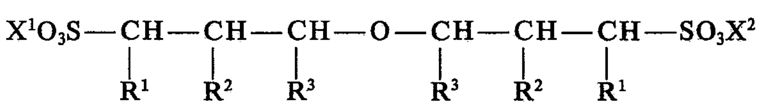

(I)

wherein

$X^1$ and $X^2$ represent H or the cation of an inorganic or organic base and
$R^1$, $R^2$ and $R^3$ represent H or methyl,

characterised in that corresponding bis-(3-mercaptopropyl)-ethers or S-substituted derivatives thereof are oxidized.

7. Process according to Claim 6, characterised in that the oxidizing agent used is nitric acid, chlorine, bromine, $H_2O_2$, permanganate, dichromate or chlorine bleach liquor.

8. Use of bis-(3-sulphopropyl)-ethers of the formula I

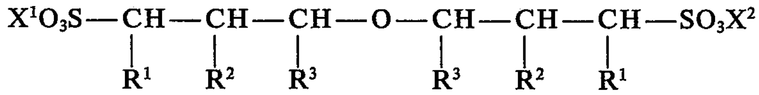

(I)

wherein

$X^1$ and $X^2$ represent H of the cation of an inorganic or organic base, and
$R^1$, $R^2$ and $R^3$ represent H or methyl,

for the sulphoalkylation of tertiary amines.

9. Use of bis-(3-sulphopropyl)-ethers of the formula I

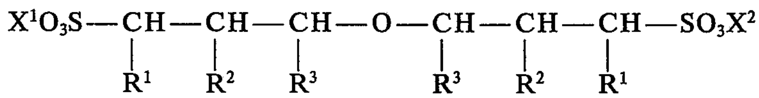

(I)

wherein

$X^1$ and $X^2$ represent H or the cation of an inorganic or organic base, and
$R^1$, $R^2$ and $R^3$ represent H or methyl,

for the sulphoalkylation of heterocyclic bases of the formula III

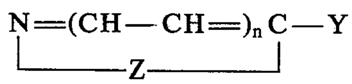

(III)

wherein

Z represents the atoms required for completing a heterocyclic group having at least one 5-membered or 6-membered heterocyclic ring,
n represents 0 or 1 and
Y represents H, halogen, a saturated or unsaturated aliphatic group, alkoxy, alkylthio or mercapto.

**Revendications**

1. Oxydes de bis-(3-sulfopropyles) de formule I:

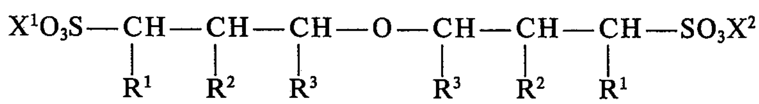

(I)

dans laquelle

$X^1$ et $X^2$ représentent H ou le cation d'une base minérale ou organique, et
$R^1$, $R^2$ et $R^3$ représentent H ou un groupe-méthyle.

2. Oxydes de bis-(3-sulfopropyles) de formule I:

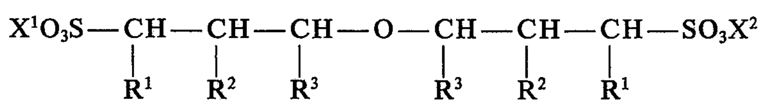

(I)

dans laquelle

$X^1$ et $X^2$ représent H, $NH_4^{\oplus}$, un cation de métal alcalin ou un cation de formule $NR_4^{\oplus}$, dans laquelle les radicaux R présents sont identiques ou différents et représentent H, un groupe alkyle ayant jusqu'à 4 atomes de carbone ou benzyle, et au moins l'un des radicaux ne représente pas H,
$R^1$ représente H ou un groupe méthyle, et
$R^2$ et $R^3$ représentent H.

3. Oxyde de bis-(3-sulfopropyle).

4. Oxyde de bis-(3-sulfobutyle).

5. Procédé de préparation d'oxydes de bis-(3-sulfopropyles) de formule I:

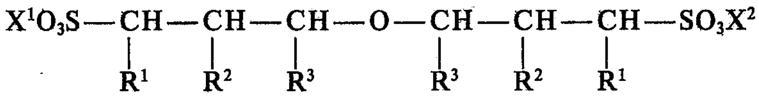

(I)

dans laquelle

$X^1$ et $X^2$ représentent H ou le cation d'une base minérale ou organique, et
$R^1$, $R^2$ et $R^3$ représentent H ou un groupe méthyle,

procédé caractérisé en ce qu'on fait réagir un oxyde de bis-(3-halogénopropyle) de formule II:

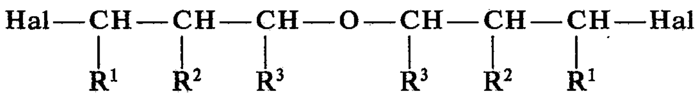

dans laquelle $R^1$, $R^2$ et $R^3$ ont le sens indiqué et Hal représente un atome d'halogène, avec un sel de l'acide sulfureux.

6. Procédé de préparation d'oxydes de bis-(3-sulfopropyles) de formule I:

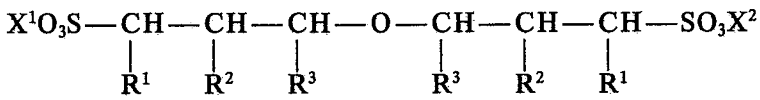

(I)

dans laquelle

$X^1$ et $X^2$ représentent H ou le cation d'une base minérale ou organique, et
$R^1$, $R^2$ et $R^3$ représentent H ou un groupe méthyle,

procédé caractérisé en ce que l'on oxyde de bis-(3-mercaptopropyle) correspondant ou des dérivés S-substitués qui en dérivent.

7. Procédé selon la revendication 6, caractérisé en ce que l'on utilise comme agent d'oxydation l'acide nitrique, le chlore, le brome, $H_2O_2$, du permanganate, du bichromate ou une solution de chlorure décolorant.

8. Utilisation d'oxydes de bis-(3-sulfopropyles) de formule I

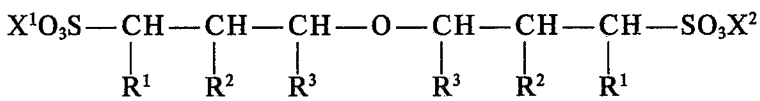

(I)

dans laquelle

$X^1$ et $X^2$ représentent H ou le cation d'une base minérale ou organique, et
$R^1$, $R^2$ et $R^3$ représentent H ou un groupe méthyle,

pour la sulfoalkylation d'amines tertiaires.

9. Utilisation d'oxydes de bis-(3-sulfopropyles) de formule I:

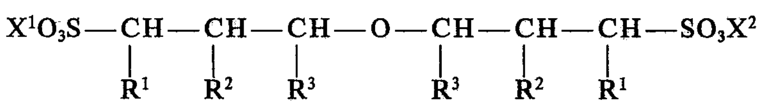

(I)

dans laquelle

$X^1$ et $X^2$ représentent H ou le cation d'une base minérale ou organique, et
$R^1$, $R^2$ et $R^3$ représentent H ou un groupe méthyle,

pour la sulfoalkylation de bases hétérocycliques de formule III:

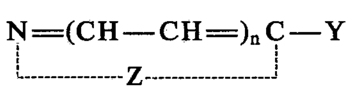

(III)

dans laquelle

Z représente les atomes nécessaires pour compléter un groupe hétérocyclique comportant au moins un noyau hétérocyclique pentagonal ou hexagonal;
n vaut 0 ou 1; et
Y représente H, un atome d'halogène, un groupe aliphatique saturé ou insaturé, un groupe alcoxy, alkylthio ou mercapto.